(19) Europäisches Patentamt
European Patent Office
Office européen des brevets

(11) Veröffentlichungsnummer: **0 005 176**
**B1**

# (12) EUROPÄISCHE PATENTSCHRIFT

(21) Anmeldenummer: **79101020.0**

(22) Anmeldetag: **04.04.79**

(51) Int. Cl.³: **C 07 C 17/156,**
C 07 C 19/045

## (54) Verfahren zur Herstellung von 1,2-Dichloräthan.

(30) Priorität: **02.05.78 DE 2819308**

(43) Veröffentlichungstag der Anmeldung:
**14.11.79 Patentblatt 79/23**

(45) Bekanntmachung des Hinweises auf die Patenterteilung:
**18.03.81 Patentblatt 81/11**

(84) Benannte Vertragsstaaten:
**BE DE FR GB IT NL SE**

(56) Entgegenhaltungen:
**DE - A - 2 718 878**
**DE - A - 2 742 409**

(73) Patentinhaber: **HOECHST AKTIENGESELLSCHAFT**
**Postfach 80 03 20**
**D-6230 Frankfurt/Main 80 (DE)**

(72) Erfinder: **Legutke, Günter, Dr.**
**Löwenburgstrasse 26**
**D-5040 Brühl (DE)**

(72) Erfinder: **Scholz, Harald, Dr.**
**Am Beissel 8**
**D-5042 Erftstadt (DE)**

(72) Erfinder: **Schuchardt, Kurt**
**Wittelsbacher Strasse 78 G**
**D-5040 Brühl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 005 176 B1

# Verfahren zur Herstellung von 1,2-Dichloräthan

Die Erfindung betrifft ein Verfahren zur Herstellung von 1,2-Dichloräthan durch Oxychlorierung von Äthylen mit Chlorwasserstoff und einem molekularen Sauerstoff enthaltenden Gas, vorzugsweise Luft, in der Gasphase bei erhöhter Temperatur in Gegenwart eines aus Kupfer-II-chlorid auf einem Träger bestehenden Katalysators im Wirbelbett, wobei man die Reaktionsgase unter Druck in zwei Kondensationsstufen abkühlt, woraufhin auskondensiertes 1,2-Dichloräthan und Wasser abgezogen werden, während nichtumgesetzte Ausgangsgase und Inertgase überwiegend im Kreislauf geführt werden.

Die bekannte Oxychlorierung erfolgt nach der Reaktionsgleichung

$$2\ C_2H_4 + O_2 + 4\ HCl \rightarrow 2\ CH_2Cl{-}CH_2Cl + 2\ H_2O$$

Die DE—B 1 618 701 beschreibt bereits ein derartiges Verfahren, bei dem 1,6 bis 2,5 Mol Äthylen mit 2,0 Mol Chlorwasserstoff und 0,6 bis 1,0 Mol Sauerstoff in Gegenwart von 0,5 bis 3,0 Mol Kohlenmonoxid an einem Kupfer-Aluminiumoxid-Katalysator umgesetzt werden, an dem gleichzeitig ein Teil des CO zu $CO_2$ oxidiert wird. Nach der Kondensation der Reaktionsprodukte müssen die nichtumgesetzten Ausgangsstoffe zur Erhaltung der Wirtschaftlichkeit im Kreislauf geführt werden. Für die Aufrechterhaltung der Wirbelbettschicht ist ein konstanter CO-Spiegel im Kreislaufgas erforderlich, der über dem besonders hergestellten Katalysator durch Oxidation von CO zu $CO_2$ und Entfernung des Kohlendioxids durch eine Wäsche mit Natronlauge erreicht wird.

Nachteilig ist bei jenem Verfahren die Einhaltung besonderer Sicherheitsvorkehrungen bei der Zugabe des reinen Sauerstoffs zwecks Vermeidung spontaner Zersetzungen infolge des hohen Gehaltes an Äthylen und Kohlenmonoxid. Weiterhin ist es nachteilig, daß das durch Oxidation gebildete Kohlendioxid aus dem Kreislaufgas ausgewaschen werden muß. Anschließend müssen Dichloräthan und andere chlorierte Kohlenwasserstoffe in gesonderten Verfahren aus dem Waschwasser entfernt werden.

In den DE—B 1 518 930, 1 518 931 und 1 518 932 sind Verfahren beschrieben, bei denen Äthylen, Sauerstoff und Chlorwasserstoff im Molverhältnis (1,02 bis 1,2):(0,5 bis 1,0):2,0 bei 200 bis 250°C und 0,7 bis 3,5 bar an einem $CuCl_2/Al_2O_3$-Katalysator im Wirbelbett umgesetzt werden. Die Reaktionsgase werden in einer 1. Kondensationsstufe auf 70 bis 100°C und in einer 2. Kondensationsstufe auf 0 bis 40°C abgekühlt. Aus den nicht kondensierbaren Gasanteilen wird restliches 1,2-Dichloräthan mit einem organischen Lösemittel herausgewaschen, aus dem in einer Desorptionskolonne Dichloräthan abgetrennt wird. Die gewaschenen Gase, die immer noch Anteile chlorierter Kohlenwasserstoffe und das organische Lösemittel enthalten, werden entweder verbrannt oder direkt ins Freie geleitet.

Die Menge des Abgases ist bei dem bevorzugten Einsatz von Luft als Sauerstoffträger so groß und der Gehalt an brennbaren Verbindungen so klein, daß Fremdheizmittel, z.B. Heizöl, aufgewendet werden müssen, um die für die Verbrennung erforderliche Temperatur einzuhalten.

Eine direkte Ableitung der Abgase ins Freie ist angesichts des Gehaltes an chlorierten und anderen Kohlenwasserstoffen, z.B. Lösemittel, aufgrund bestehender Gesetze heute kaum noch zu verwirklichen.

Neben diesen Schwierigkeiten bei der Handhabung so großer Abgasmengen treten zwangsweise auch Verluste an Äthylen, Dichloräthan und organischen Lösemitteln auf.

Schließlich beschreibt die DE—A 2 626 133 ein zyklisches Verfahren zur Herstellung von 1,2-Dichloräthan durch Oxyhydrochlorierung von Äthylen, bei dem 80 bis 98 Volumen% der nicht kondensierbaren Gase ohne Wäsche im Kreislauf geführt werden. Das Kreislaufgas enthält je etwa 0,1 bis 10 Volumen% Äthylen und Sauerstoff und weniger als 20 Volumen% 1,2-Dichloräthan. 2 bis 20 Volumen% des Kreislaufgases werden jeweils aus dem System ausgeschleust. Die Reaktionsgase werden nacheinander in einem Kühlturm auf 82 bis 121°C, in einem 1. Kondensator auf 32 bis 49°C und in einem 2. Kondensator auf 27 bis 38°C abgekühlt, um Dichloräthan und Wasser im wesentlichen daraus zu entfernen. Als Oxidationsgas wird bei diesem Verfahren im wesentlichen reiner Sauerstoff verwendet.

Die Sauerstoffzugabe erfolgt beim Verfahren der DE—A 2 626 133 mit dem Strom der Ausgangsstoffe unmittelbar in die Reaktionszone und stellt damit ein Sicherheitsrisiko dar, da ein Zusammentreffen von im wesentlichen reinem Sauerstoff und Äthylen kaum zu vermeiden ist. Die Zugabe von im wesentlichen reinem Sauerstoff zum Kreislaufgas vor dem Reaktor ist bei der im Beispiel der DE—A 2 626 133 angegebenen Gaszusammensetzung der Kreislaufgases wegen der Zündgefahr auch nicht möglich. Die untere Explosionsgrenze derartiger Gasgemische liegt sehr niedrig, und zwar je nach dem Sauerstoffgehalt im Gasgemisch bei 3,0 bis 6,0 Vol%, gerechnet als Summe der brennbaren Gase Äthylen, 1,2-Dichloräthan, Kohlenmonoxid und organischen Nebenprodukte. Aus dieser niedrigen Explosionsgrenze der Stoffe Äthylen, Kohlenmonoxid, 1,2-Dichloräthan und organische Nebenprodukte ergeben sich erhebliche Schwierigkeiten, die wohl auch der Grund dafür sind, daß alle bisherigen Kreislaufgasverfahren technisch keine Bedeutung erlangt haben.

Die Nachteile der vorbeschriebenen Verfahren werden durch die in den DE—A 27 18 878 und 27 42 409 beschriebenen Verfahren überwunden. Gegenstand dieser Patente sind Verfahren zur Herstellung von 1,2-Dichloräthan durch Oxychlorierung von Äthylen mit Chlorwasserstoff und einem molekularen Sauerstoff enthaltenden Gas, vorzugsweise Luft, in der Gasphase bei erhöhter Temperatur in Gegenwart eines aus Kupfer-II-chlorid auf einem Träger bestehenden Katalysators im Wirbelbett, wobei man die Reaktionsgase unter Druck in zwei Kondensationsstufen abkühlt, woraufhin auskondensiertes 1,2-Dichloräthan und Wasser abgezogen werden, während nichtumgesetzte Ausgangsgase und Inertgase überwiegend im Kreislauf geführt werden, welche grundsätzlich dadurch gekennzeichnet sind, daß man die Reaktionsgase in einer 3. Kondensationsstufe unter Druck nur soweit auf eine zwischen 5 und 18°C liegende Temperatur abkühlt, daß noch 0,5 bis 3 Volumen%, vorzugsweise 0,5 bis 1,5 Volumen%,1,2-Dichloräthan im Kreislaufgas verbleiben, und daß man den verbrauchten Sauerstoff durch Zuleiten reinen Sauerstoffs direkt zum Kreislaufgas vor dem Reaktor ergänzt.

Weitere Merkmale der Verfahren der DE—A 27 18 878 und 27 42 409 bestehen darin, daß man

a) den Gesamtgehalt des Kreislaufgases an den brennbaren Komponenten Äthylen, 1,2-Dichloräthan, Kohlenmonoxid und organischen Nebenprodukten auf Werte unterhalb der zwischen 3 und 6 Volumen% (bei Sauerstoffgehalten von 25 bis 12 Vol%) liegenden unteren Explosionsgrenze einstellt, indem man

   1. die Ausgangsstoffe Äthylen, Sauerstoff und Chlorwasserstoff im Molverhältnis (1,00 bis 1,10):(0,50 bis 0,70): 2,00 einsetzt,

   2. Kohlenmonoxid am Katalysator zu 50 bis 100, vorzugsweise 60 bis 90, Mol% zu Kohlendioxid oxydiert und

   3. den erforderlichen Gehalt des Kreislaufgases an Inertgas durch Zufuhr der berechneten Menge Luft und/oder Inertgas aufrechterhält;

b) die brennbaren Komponenten Äthylen, 1,2-Dichloräthan, Kohlenmonoxid und organische Nebenprodukte in an sich bekannter Weise mit üblichen Absorptionsmitteln einzeln oder gemeinsam, teilweise oder vollständig, aus dem Kreislaufgas vor Zugabe des reinen Sauerstoffs entfernt;

c) durch Konstanthalten der Menge des Kreislaufgases eine gleichmäßige Durchwirbelung des Katalysatorbettes gewährleistet;

d) die Katalysatorleistung durch Veränderung des Sauerstoffgehaltes im Kreislaufgas vor dem Reaktor zwischen 12 und 25 Vol% steuert;

e) die Zusammensetzung des Kreislaufgases nach der Sauerstoffzugabe vor dem Reaktor laufend analytisch überwacht;

f) die Reaktionsgase unter Druck in einer 1. Kondensationsstufe auf 70 bis 100°C und in einer 2. Kondensationsstufe auf 37 bis 40°C abkühlt.

Im einzelnen umfassen die DE—A 27 18 878 und 27 42 409 auch eine spezielle Arbeitsweise zur Herstellung von 1,2-Dichloräthan durch Oxychlorierung von Äthylen mit Chlorwasserstoff und einem molekularen Sauerstoff enthaltenden Gas, vorzugsweise Luft, in der Gasphase bei Temperaturen von 200 bis 250°C in Gegenwart eines aus Kupfer-II-chlorid auf einem Träger bestehenden Katalysators im Wirbelbett, wobei man die Reaktionsgase unter Druck in einer 1. Kondensationsstuffe auf 70 bis 100°C und in einer 2. Kondensationsstufe auf 0 bis 40°C abkühlt, woraufhin auskondensiertes 1,2-Dichloräthan und Wasser abgezogen werden, während nichtumgesetzte ausgangsgase und Inertgase überwiegend im Kreislauf geführt werden, welche dadurch gekennzeichnet ist, daß man die Reaktionsgase in der 2. Kondensationsstufe auf 37 bis 40°C und in einer 3. Kondensationsstufe unter Druck nur soweit auf eine zwischen 5 und 18°C liegende Temperatur abkühlt, daß noch 0,5 bis 3 Volumen%, vorzugsweise 0,5 bis 1,5 Volumen%, 1,2 Dichloräthan im Kreislaufgas verbleiben, und daß man den verbrauchten Sauerstoff durch Zuleiten reinen Sauerstoffs direkt zum Kreislaufgas vor dem Reaktor ergänzt.

Das molare Verhältnis der Ausgangsgase Äthylen:Sauerstoff:Chlorwasserstoff beträgt vorzugsweise (1,00 bis 1,04):(0,50 bis 0,60):2,00. Die Umsetzung wird vorzugsweise bei einem Druck von 0,7 bis 3,5 bar durchgeführt. Derselbe Druckbereich ist dann auch bei den drei Kondensationsstufen wirksam. Als Katalysator wird beispielsweise der in der DE—B 1 518 932 beschriebene $CuCl_2/Al_2O_3$-Katalysator verwendet. Das Kreislaufgas wird nicht mit Natronlauge gewaschen, sondern direkt in den Reaktor zurückgeleitet.

Die in den DE—A 27 18 878 und 27 42 409 erläuterten Verfahrensweisen sehen eine Kreislaufführung des von 1,2-Dichloräthan befreiten Reaktionsgases sowie die Ergänzung des bei der Reaktion verbrauchten Sauerstoffs durch Zugabe von reinem Sauerstoff in das Kreislaufgas vor. Unter Einhaltung der für diese Verfahrensweisen gegebenen Betriebsbedingungen ist an sich gewährleistet, daß nach

Zuführung des reinen Sauerstoffs zum Kreislaufgas sowie nach anschließender erwärmung des Sauerstoff/Kreislaufgas-Gemisches auf etwa 150°C die unteren, niedrigen Explosionsgrenzen des Gasgemisches nicht erreicht werden. Das diese Grenzen niedrig liegen, kann man den Vorteil, den höhere Konzentrationen von 1,2-Dichloräthan im Kreislaufgas vor Eintritt des Gases in den Reaktor bieten, nur beschränkt ausnutzen. Es wird daher bereits im DE—A 27 42 409 die Wäsche des Kreislaufgases mit aromatischen Kohlenwasserstoffen vorgeschlagen. Auch ist es bei den gegebenen niedrigen Explosionsgrenzen nicht möglich, durch Erhöhung der Sauerstoffmenge im Kreislaufgas den Umsatz der Reaktionskomponenten im Reaktor zu erhöhen, da hierdurch rascher die kritische Zusammensetzung des Kreislaufgases erreicht würde.

Es wurde nunmehr gefunden, daß eine Änderung der Zuführungsstellen des Chlorwasserstoffs und des reinen Sauerstoffs gegenüber den beiden Verfahren der DE—A 27 18 878 und 27 42 409 sich auf die Verfahren und deren Sicherheit vorteilhaft auswirkt.

Somit betrifft die Erfindung gegenüber den Verfahren der DE—A 27 18 878 und 27 42 409 ein verbessertes Verfahren zur Herstellung von 1,2-Dichloräthan durch Oxychlorierung von Äthylen mit Chlorwasserstoff und einem molekularen Sauerstoff enthaltenden Gas, wobei man den vorerwärmten Chlorwasserstoffstrom ganz oder teilweise in den auf 150—200°C erhitzten Kreislaufgasstrom einleitet und anschließend reinen Sauerstoff dem Kreislaufgasstrom zwischen der Aufgabestelle des Chlorwasserstoffs und der Eintrittsstelle des Kreislaufgases in den Reaktor zuführt.

Der Sauerstoff wird dem Kreislaufgasstrom im allgemeinen mit einer Temperatur von 20 bis 150°C zugeführt, während der Chlorwasserstoffstrom auf eine Temperatur von 150—200°C vorerwärmt ist.

Nach einer bevorzugten Ausführungsform des Verfahrens der Erfindung wird der reine Sauerstoff dem Kreislaufgasstrom bis zu einem Gehalt von 10 bis 30 Vol%, bezogen auf die Gesamtgasmenge aus Kreislaufgas, Chlorwasserstoff und Sauerstoff zugeführt.

Durch die erfindungsgemäße Zuführung des Chlorwasserstoffes in das Kreislaufgas wird der Reaktionsablauf der Reaktionspartner im Fließbettkatalysator nicht verändert. Andererseits wird durch vorerwähnte Maßnahme die Konzentration sämtlicher am Kreislaufgasstrom beteiligten Gase sowie brennbaren Substanzen vermindert und damit das Explosionsrisiko des Gasgemisches herabgesetzt. Weiterhin bietet die erfindungsgemäße Verfahrensweise den Vorteil, die Menge des zuzuführenden reinen Sauerstoffs erhöhen zu können und dadurch bei bestehenden Anlagen eine Kapazitätserweiterung zu erreichen. Durch die Steigerung der Sauerstoffmenge wird die Konzentration des Sauerstoffs in dem Gemisch aus Kreislaufgas und Chlorwasserstoff nur unwesentlich erhöht, da Sauerstoff und Chlorwasserstoff per se in bestimmtem Molverhältnis zugesetzt werden. Die in älteren Verfahren praktizierte Vorerhitzung des reinen Sauerstoffs vor der Vereinigung mit dem Kreislaufgas erübrigt sich beim Verfahren der erfindung, indem der Sauerstoff dem Gemisch aus Kreislaufgas und Chlorwasserstoff nunmehr kalt zugegeben werden kann, wenn die genannten Gemischkomponenten entsprechend vorerwärmt wurden. Der Mischprozeß erfolgt in bekannter Weise, z.B. mit Hilfe einer Venturidüse.

Das Verfahren der Erfindung wird nachstehend anhand der beigefügten Zeichnung näher erläutert:

Über Leitung 1 und Vorwärmer 2 wird Äthylen dem Wirbelbettreaktor 7 zugeführt. Gleichzeitig wird entweder ein Teil einer vorbestimmten Menge von Chlorwasserstoffgas über Leitung 3 und Vorwärmer 4 nach Mischung mit dem Äthylengasstrom in den Reaktor 7 eingeleitet und die Restmenge des Chlorwasserstoffgases über die Leitung 30 mit dem Kreislaufgas der Leitung 25 vermischt oder die Gesamtmenge des Chlorwasserstoffs über die Leitungen 3 und 30 dem Kreislaufgas der Leitung 25 zugeführt. Uber Leitung 5 und Vorwärmer 6 wird Luft in den Reaktor 7 geleitet. Im Reaktor 7 befindet sich ein Kupfer-II-chlorid-Katalysator. Die Oxychlorierung ist exotherm, die Temperatur wird durch einen Heißwasserkreislauf z.B. auf 220 bis 235°C gehalten. Der Druck im gesamten System beträgt dabei etwa 3 bar. Über einen Zyklon gelangt das Gas über Leitung 8 in die erste Kondensationsstufe 9, wo das Gas mit Reaktionswasser aus Trennbehälter 12 über Pumpe 17 und Leitung 18 auf etwa 80°C gekühlt wird. Dabei kondensieren nichtverbrauchter Chlorwasserstoff und die größte Menge an Reaktionswasser. Über Leitung 10 und Kühler 11 (2. Kondensationsstufe) wird das Gas auf etwa 40°C gekühlt.1,2-Dichloräthan und restliches Wasser kondensieren und werden im Trenngefäß 12 grob getrennt. Das Wasser wird zur Kondensationsstufe 9 zurückgeführt und über Leitung 26 zur Aufarbeitung abgezogen. Rohdichloräthan wird aus dem Trenngefäß 12 über Leitung 15 und Pumpe 16 zur Reinigungsstufe abgezogen. Die nicht kondensierten Gasanteile werden im Kühler 13 (3. Kondensationsstufe) soweit auf eine zwischen 5 und 18°C liegende Temperatur gekühlt, daß 0,5 bis 3 Volumen% 1,2-Dichloräthan im Gas verbleiben. Im Abscheider 14 wird nachkondensiertes 1,2-Dichloräthan aufgefangen und zum Trenngefaß 12 zurückgeleitet. Das verbleibende Gas wird über Leitung 20, Kompressor 21, Leitung 22, Vorheizer 24 und Leitung 25 zum Reaktor 7 zurückgeführt. Vor Eintritt des Gemisches aus vorerhitztem Kreislaufgas und Chlorwasserstoffgas über Leitung 25 in den Reaktor 7 wird über Leitung 23 dem

Gemisch in Leitung 25 soviel Sauerstoff mit einer Temperatur von 20 bis 150°C unter intensiver Durchmischung zugeführt, daß die Gesamtmenge $O_2$ im Gemisch von Chlorwasserstoff, Kreislaufgas und $O_2$ 10 bis 30 Vol% beträgt. Nach Kompressor 21 kann auch noch eine Absorptionsanlage 27 mit flüssigen oder festen Absorptionsmitteln zwischengeschaltet werden, über die man dann zweckmäßig das gesamte nicht kondensierbare Gas leitet. Die Gaszusammensetzung wird nach der Sauerstoffzugabe vor dem Reaktor laufend analytisch überwacht, um das Auftreten zündfähiger Gemische im Kreislaufgas zu vermeiden. Nach Erreichen der notwendigen Gasmenge wird die Zuführung von Luft über Leitung 5 eingeschränkt, d.h. es wird nur noch soviel Luft zugeführt, daß der Stickstoffgehalt im System in etwa konstant bleibt. Zur Konstanthaltung der Gasmengen wird über Leitung 19 bzw. 28 die Menge an Gas abgenommen, die der Verbrennung von Äthylen zu CO und $CO_2$ entspricht, und in an sich bekannter Weise aufgearbeitet. Über Leitung 29 kann Inertgas zugesetzt werden, wobei die Zuführung des Inertgases in Abhängigkeit von der analytisch überwachten Gaszusammensetzung automatisch geregelt werden kann.

Die in den folgenden Beispielen beschriebenen Ergebnisse wurden in einem Wirbelbettreaktor mit einem Durchmesser von 3,0 m und einer Höhe von 29,9 m erzielt. Als Katalysator wurde Kupfer-II-chlorid, aufgetragen auf Aluminiumoxid, mit etwa 4 Gewichts% Kupfer eingesetzt. Die Katalysatormenge betrug im Durchschnitt 48 700 kg.

Beispiel 1

Äthylen, Chlorwasserstoff und Sauerstoff (in Form von Luft) wurden im Molverhältnis von 1,04:2,00:0,55 eingesetzt, jeweils auf 145—150°C vorerwärmt und dann dem Verteilerboden des Reaktors zugeführt. Die Reaktionskomponenten setzten sich am Katalysator unter Wärmeentwicklung überwiegend zu 1,2-Dichloräthan um. Die freiwerdende Reaktionswärme wurde mit Hochdruckwasserkühlung unter Dampfgewinnung abgeführt. Die Temperatur im Reaktor betrug 223°C, der Druck 3 bar. Durch 3stufige Kondensation wurde das Reaktionsgas abgekühlt und Rohdichloräthan und Wasser kondensiert, wobei in der 3. Stufe auf etwa 17°C gekühlt wurde. Das Abgas der 3. Kondensationsstufe besaß folgende Zusammensetzung in Volumen%:

| | |
|---|---|
| Sauerstoff | 8,0 |
| Stickstoff | 33,3 |
| Kohlenmonoxid | 2,5 |
| Kohlendioxid | 53,0 |
| 1,2-Dichloräthan | 2,1 |
| Äthylen | 0,62 |
| sonstige brennbare Bestandteile | <0,5 |

Die Summe aller brennbaren Bestandteile des Gasgemisches betrug 5,72 Vol%. Dieses Gasgemisch (Kreislaufgas) wurde auf 5,5 bar komprimiert, auf 166°C erwärmt und über Leitung 30 mit auf 166°C erwärmtem Chlorwasserstoff vermischt. Über eine Mischeinrichtung bekannter Bauart wurde dem Gasgemisch (Kreislaufgas-Chlorwasserstoff) über Leitung 23 reiner Sauerstoff mit einer Temperatur von 20°C zugemischt. Chlorwasserstoff und Sauerstoff wurden dabei wiederum im Molverhältnis von 2,00:0,55 zudosiert. Vor Eingang in den Reaktor besaß das Gasgemisch Kreislaufgas-Chlorwasserstoff-Sauerstoff eine Temperatur von 150°C, die Summe der brennbaren Substanzen im Gemisch war auf 3,25 Vol% abgesunken und der Sauerstoffgehalt des Gemisches betrug 13,8 Vol%.

Der Äthylenumsatz betrug 99,82%, die Ausbeute an 1,2-Dichloräthan 96,72% der Theorie, bezogen auf die eingesetzte Menge an Äthylen. Die Kontaktleistung betrug 280 g 1,2-Dichloräthan je kg Katalysator und Stunde.

Beispiel 2

Es wurde analog Beispiel 1 verfahren, wobei die über Leitung 30 zugeführte Menge an Chlorwasserstoff vorab um 30 Vol% erhöht wurde, woraufhin anschließend die über Leitung 23 zudosierte Menge an Sauerstoff ebenfalls um 30 Vol% gefahrlos erhöht werden konnte. Vor Eingang in den Reaktor besaß das Gasgemisch eine Temperatur von 150°C, die Summe der brennbaren Substanzen betrug 2,9 Vol% und der Sauerstoffgehalt des Gemisches war auf 14,7 Vol% angestiegen. Um das Molverhältnis Äthylen / Chlorwasserstoff / Sauerstoff = 1,04:2,00:0,55 einzuhalten, wurde die über Leitung 1 zudosierte Menge Äthylen schließlich auch um 30 Vol% erhöht.

Der Äthylenumsatz betrug 99,84%, die Ausbeute an 1,2-Dichloräthan, bezogen auf den Äthylenumsatz, 96,42% der Theorie. Die Kontaktleistung betrug 360 g/kg Katalysator und Stunde.

**Patentansprüche**

1. Verfahren zur Herstellung von 1,2-Dichloräthan durch Oxychlorierung von Äthylen mit Chlorwasserstoff und einem molekularen Sauerstoff enthaltenden Gas, vorzugsweise Luft, in der Gasphase bei erhöhter Temperatur in Gegenwart eines aus Kupfer-II-chlorid auf einem Träger bestehenden Katalysators im Wirbelbett, wobei man die Reaktionsgase unter Druck in zwei Kondensationsstufen abkühlt, auskondensiertes 1,2-Dichloräthan und Wasser abzieht, während nichtumgesetzte Ausgangsgase und Inertgase überwiegend im Kreislauf geführt werden, derart, daß man die Reaktionsgase der 2. Kondensationsstufe in einer 3. Kondensationsstufe unter Druck nur soweit auf eine zwischen 5 und 18°C liegende Temperatur abkühlt, daß noch 0,5 bis 3 Volumen%, vorzugsweise 0,5 bis 1,5 Volumen%, 1,2-Dichloräthan im Kreislaufgas verbleiben, und daß man

den verbrauchten Sauerstoff durch Zuleiten reinen Sauerstoffs direkt zum Kreislaufgas vor dem Reaktor ergänzt, dadurch gekennzeichnet, daß man den vorerwärmten Chlorwasserstoffstrom ganz oder teilweise in den auf 150—200°C erhitzten Kreislaufgasstrom einleitet und anschließend den reinen Sauerstoff dem Kreislaufgasstrom zwischen der Aufgabestelle des Chlorwasserstoffs und der Eintrittsstelle des Kreislaufgases in den Reaktor zuführt.

2. Verfahren nach Anspruch 1, dadurch gekennzeichnet, daß man den reinen Sauerstoff dem Kreislaufgasstrom mit einer Temperatur von 20 bis 150°C zuführt.

3. Verfahren nach Anspruch 1 oder 2, dadurch gekennzeichnet, daß man den Chlorwasserstoffstrom auf eine Temperatur von 150—200°C vorerwärmt.

4. Verfahren nach Anspruch 1—3, dadurch gekennzeichnet, daß der reine Sauerstoff dem Kreislaufgasstrom bis zu einem Gehalt von 10 bis 30 Vol%, bezogen auf die Gesamtgasmenge aus Kreislaufgas, Chlorwasserstoff und Sauerstoff, zugeführt wird.

**Claims**

1. Process for making 1,2-dichloroethane by subjecting ethylene to an oxychlorination reaction with hydrogen chloride and a gas containing molecular oxygen, preferably air, in gas phase, at elevated temperature and in contact with a fluidized bed catalyst of copper-II-chloride on a carrier, wherein the reaction gases are cooled under pressure in two condensation stages, condensed 1,2-dichloroethane and water are removed, the bulk of unreacted starting gas and inert gas are recycled, the reaction gases coming from the second condensation stage are delivered to a third condensation stage, cooled therein under pressure down to a temperature within the range 5 to 18°C and to the extent necessary to retain in the recycle gas 0.5 to 3 volume %, preferably 0.5 to 1.5 volume %, of 1,2-dichloroethane, and the recycle gas is admixed, directly upstream of the reactor, with a quantity of pure oxygen necessary to replace consumed oxygen, which comprises: introducing preheated hydrogen wholly or partially into the recycle gas heated to 150 to 200°C and then admixing the recycle gas with pure oxygen, the oxygen being admitted at a point lying between the hydrogen chloride feet point and the feed point of the recycle gas into the reactor.

2. Process as claimed in claim 1, wherein the pure oxygen admitted to the recycle gas has a temperature of 20 to 150°C.

3. Process as claimed in claim 1 or 2, wherein the hydrogen chloride is preheated to a temperature of 150 to 200°C.

4. Process as claimed in claim 1, wherein the recycle gas is admixed with a quantity of pure oxygen necessary for it to contain 10 to 30 volume % of $O_2$, based on the overall quantity of recycle gas, hydrogen chloride and oxygen.

**Revendications**

1. Procédé de préparation du 1,2-dichloréthane par oxychloration de l'éthylène à l'aide du chlorure d'hydrogène et d'un gaz contenant de l'oxygène moléculaire, de préférence l'air, en phase gazeuse, à température élevée, en présence d'un catalyseur consistant en chlorure de cuivre-II sur un support en lit tourbillonnaire, dans lequel on refroidit les gaz de réaction sous pression dans deux étages de condensation, on évacue le 1,2-dichloréthane condensé et l'eau, et on recycle la plus grande partie des gaz de départ non convertis et des gaz inertes, les gaz de réaction sortant du deuxième étage de condensation étant refroidis dans un troisième étage de condensation sous pression à une température de 5 à 18°C dans des conditions telles qu'il subsiste encore de 0,5 à 3% en volume, de préférence de 0,5 à 1,5% en volume de 1,2-dichloréthane dans les gaz recyclés, et l'oxygène consommé étant remplacé par introduction d'oxygène pur directement dans les gaz de recyclage avant le réacteur, caractérisé en ce que le courant de chlorure d'hydrogène réchauffé au préalable est injecté en totalité ou en partie dans le courant des gaz de recyclage chauffé à 150—200°C et l'oxygène pur est ensuite introduit dans le courant des gaz de recyclage entre l'endroit d'introduction du chlorure d'hydrogène et l'endroit où les gaz de recyclage entrent dans le réacteur.

2. Procédé selon la revendication 1, caractérisé en ce que l'on envoie l'oxygène pur dans le courant des gaz de recyclage à une température de 20 à 150°C.

3. Procédé selon la revendication 1 où 2, caractérisé en ce que le courant de chlorure d'hydrogène est réchauffé au préalable à une température de 150 à 200°C.

4. Procédé selon l'une quelconque des revendications 1 à 3, caractérisé en ce que l'oxygène pur est introduit dans le courant des gaz de recyclage jusqu'à une teneur de 10 à 30% en volume, par rapport à la quantité totale des gaz constitués des gaz de recyclage, de chlorure d'hydrogène et d'oxygène.

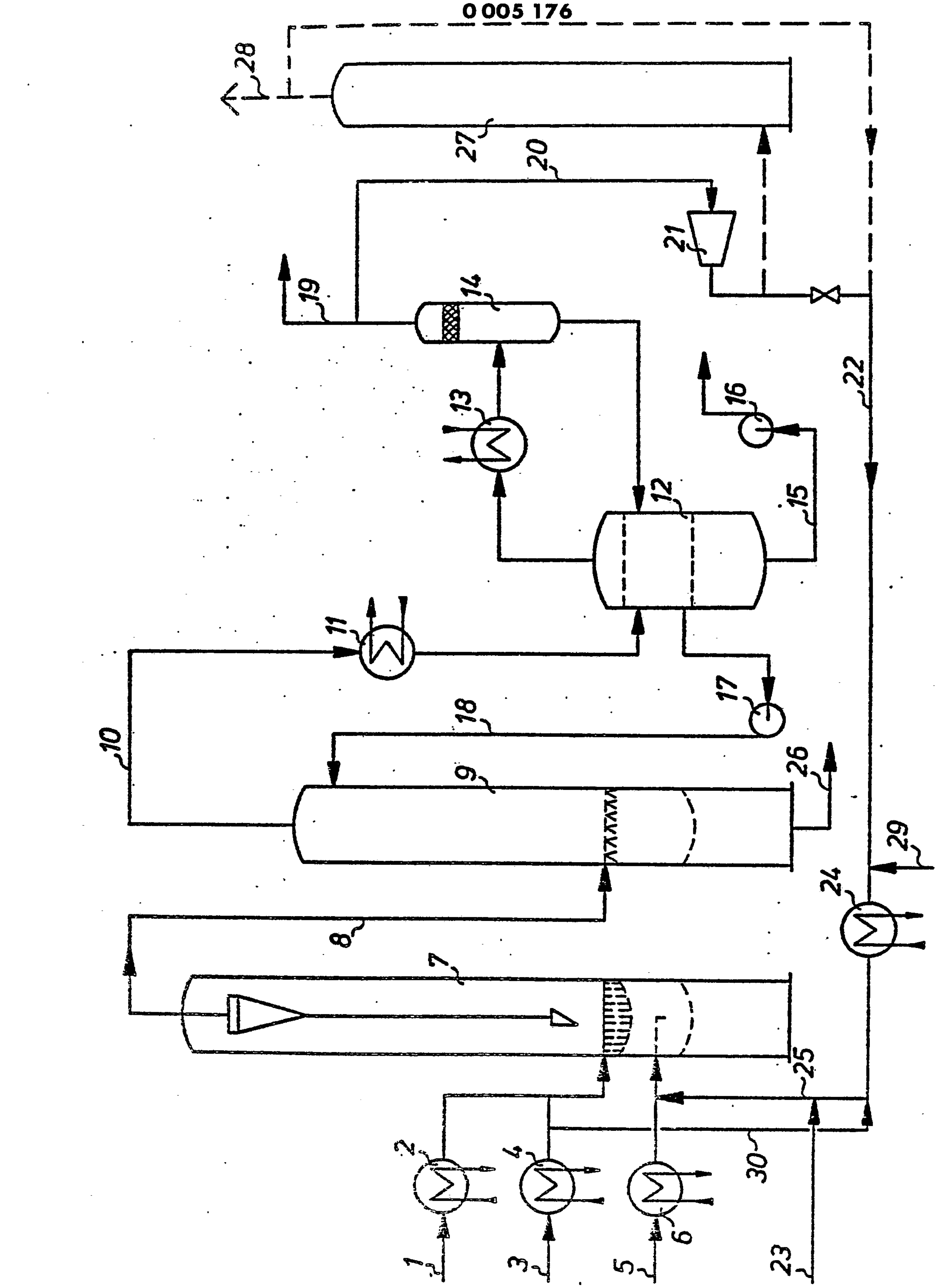
1
2
3
4
5
6
7
8
9
10
11
12
13
14
15
16
17
18
19
20
21
22
23
24
25
26
27
28
29
30

1